# DEMANDE DE BREVET EUROPEEN

(11) **EP 4 115 896 A1**
(43) Date de publication de la demande: **11.01.2023**
(21) Numéro de dépôt: 22166269.5
(22) Date de dépôt: 28.07.2017
(51) Int. Cl.: A61K 36/88, A61P 15/10, A61P 25/24, A61K 8/97

(54) **EXTRAIT DE PLANTE TRÈS CONCENTRÉ EN SAFRANAL, PROCÉDÉ D'OBTENTION ET UTILISATIONS**

(30) Priorité: 28.07.2016 FR 1657297
(62) Demande divisionnaire de: 17754296.6
(71) Demandeur: Activ'inside, 33750 Beychac et Caillau (FR)
(72) Inventeur: GAUDOUT, David, 33360 CARIGNAN DE BORDEAUX (FR); REY, Stéphane, 26200 MONTELIMAR (FR); LEMAIRE, Benoit, 33500 LIBOURNE (FR); MORAS, Benjamin, 33100 BORDEAUX (FR); DUMOULIN, Marion, 49100 ANGERS (FR)
(74) Mandataire: Aquinov

(57) **Abrégé**

L'objet de l'invention est un extrait végétal comprenant une concentration mesurée par méthode HPLC d'au moins 0,2% de safranal en poids par rapport au poids total de la matière sèche. L'invention a également pour objet un procédé d'obtention d'un tel extrait, les compositions l'incluant et son utilisation.

## Description

La présente invention se rapporte à un nouvel extrait obtenu à partir d'une matière première végétale contenant du safranal, notamment du safran, ledit extrait présentant une concentration en safranal plus importante que les extraits végétaux connus actuellement. L'invention a également pour objet un procédé particulier permettant d'obtenir un tel extrait, ainsi que les compositions l'incluant et ses utilisations.

Le safranal peut être extrait de plusieurs plantes telles que *Crocus sativus, Centaurea sibthorpii, Centaurea consanguinea, Centaurea amanicola, Erodium cicutarium, Chinese green tea, Calycopteris floribunda, Crocus heuffelianus, Sambucus nigra, Gardenia jasminoides, Citrus limon, Cuminum cyminum L., Achillea distans* mais est majoritairement extrait du *Crocus sativus* connu également sous le nom de safran.

La safran est une épice traditionnelle, essentiellement cultivée en Iran, utilisée dans de nombreuses applications telles que les troubles de l'humeur, le syndrome prémenstruel, les troubles de l'érection ou encore la beauté de la peau tel que cela est présenté notamment dans Javadi B & al. « A survey on saffron in major islamic traditional medicine books » Iranian journal of basic medical sciences 2013; 16(1): 1-11. Plusieurs études cliniques cherchant à démontrer l'efficacité du safran ont été réalisées ces dernières années, en particulier en ce qui concerne le soulagement des troubles dépressifs et anxieux comme c'est le cas par exemple *dans :* Akhondzadeh S & al. « Comparison of Crocus sativus L. and imipramine in the treatment of mild to moderate depression: a pilot double-blind randomized trial » [ISRCTN45683816]. BMC Complément Altern Med 2004; 4: 12*,* ou plus récemment dans : Hausenblas HA & al. « Saffron (Crocus sativus L.) and major depressive disorder: a meta-analysis of randomized clinical trials». Journal of integrative medicine 2013; 11(6): 377-83*.*

Le safran *(Crocus sativus)* comprend plusieurs molécules, dont le safranal, molécule volatile responsable de l'odeur de l'épice, reconnue pour son efficacité dans les applications précitées. Les extraits utilisés dans l'art antérieur sont pour la plupart standardisés à 2% de safranal par spectrométrie UV selon la norme ISO 3632-2 :2010 *(*Spices - Saffron (Crocus sativus L.) - Part 2: Test methods. ISO International standard; 2010: 1-42). Cette norme décrit un protocole d'analyse très global consistant à dissoudre l'extrait de safran dans l'eau, l'agiter, le filtrer puis effectuer une lecture spectrophotométrique à 330nm. Toutefois, cette méthode est très critiquable car l'absence d'utilisation de solvants et/ou de réactifs spécifiques entraine la quantification de molécules autres que le safranal si bien qu'elle ne reflète pas du tout la concentration réelle en safranal de l'extrait.

En effet, l'analyse par HPLC (aussi appelée CLHP : Chromatographie Liquide Haute Performance) du safranal dans les extraits de l'art antérieur révèle des concentrations réelles de 30 à 1000 fois inférieures à celles obtenues par l'analyse par spectrométrie UV selon la norme ISO 3632-2, et avec une variabilité très importante dans la corrélation des deux méthodes, comme le démontrent les résultats du Tableau 1 ci-dessous :

**Tableau 1 : comparaison des résultats obtenus avec deux méthodes de dosage de safranal dans plusieurs extraits de safran de l'art antérieur (résultats donnés en pourcentage en poids par rapport au poids total de la matière sèche de l'extrait).**

| Extrait de safran | Safranal Méthode ISO 3632-2:2010 | Safranal Méthode HPLC |
|---|---|---|
| Saffron dry extract - Natac | 2.71 | 0.0650 |
| Safran ES stigmate - Plantex | 2.12 | 0.0040 |
| Saffr'Activ^{®} - Green Plant Extract | 2.32 | 0.0380 |
| Affron^{®} - Pharmactive | 2.90 | 0.0190 |

En réalité, les extraits de plantes contenant du safranal, et en particulier les extraits de safran, connus obtenus avec les procédés de l'art antérieur, sont très peu dosés en safranal.

La méthode de mesure est donc importante, car elle conduit à des résultats différents de ceux obtenus avec la méthode de mesure antérieure par spectrométrie utilisée systématiquement pour mesurer la concentration en safranal dans un produit. Cette méthode antérieure, référencée IS03632-2 : 2010, surévalue la teneur en safranal, comme démontré dans la présente demande ainsi que dans la publication Garcia-Rodriguez et al., 2017 « Comparative evaluation of an ISO 3632 method and an HPLC-DAD method for safranal quantity determination in saffron ». Dans cette publication, l'étude a été réalisée sur 390 échantillons de safran. La surestimation peut-être de 20 à 50 fois. Ainsi, un extrait titré à X% en safranal par la méthode de mesure de référence ISO3632-2 : 2010, comprend en réalité beaucoup moins de safranal.

L'objectif de la présente invention est de répondre à cette problématique en proposant un extrait de safran plus fortement dosé en safranal que les extraits existants.

En particulier, l'invention vise un extrait végétal obtenu à partir de safran, comprenant une concentration mesurée par méthode HPLC d'au moins 0,2% de safranal en poids par rapport au poids total de la matière sèche de l'extrait.

Cet extrait peut notamment être obtenu par un procédé comprenant une étape de traitement thermique, en particulier un procédé spécifique qui comprend la mise en œuvre des étapes suivantes :
- Eventuellement séchage de la matière première,
- Broyage de la matière première séchée,
- Extraction aqueuse ou hydroalcoolique ou avec un solvant organique,
- Imprégnation sur un support de l'extrait obtenu,
- Traitement thermique de l'extrait.

Ce procédé est économique, simple à mettre en œuvre et permet d'obtenir un extrait de safran, comprenant une concentration mesurée par méthode HPLC d'au moins 0,2% de safranal en poids par rapport au poids total de la matière sèche de l'extrait. Avantageusement, un tel extrait contient une concentration réelle en safranal supérieure à celle des extraits connus actuellement et présente ainsi une efficacité plus importante en particulier pour lutter contre la dépression, l'anxiété, les troubles de l'humeur, les troubles de l'érection et les troubles prémenstruels. L'invention a donc aussi pour objet l'extrait de safran pour ces utilisations, et vise également les compositions cosmétiques, alimentaires, nutritionnelles ou médicamenteuses l'incluant.

D'autres caractéristiques et avantages ressortiront de la description en détails de l'invention qui va suivre, en regard des figures annexées qui représentent :
- Figure 1 : chromatogramme de l'extrait selon l'invention de l'exemple 1, obtenu par méthode UHPLC,
- Figure 2 : chromatogramme de l'extrait selon l'invention de l'exemple 2 obtenu par méthode UHPLC.
- Figure 3A : chromatogramme d'un extrait de l'art antérieur selon l'exemple 3A (obtenu sans traitement thermique) obtenu par méthode UHPLC,
- Figure 3B : chromatogramme d'un extrait de l'art antérieur selon l'exemple 3B (obtenu sans traitement thermique) obtenu par méthode UHPLC,

L'invention a donc pour objet un extrait végétal obtenu à partir d'une plante (ou matière première végétale) contenant du safranal, comprenant une concentration mesurée par méthode HPLC d'au moins 0,2% de safranal en poids par rapport au poids total de la matière sèche.

Par « extrait végétal obtenu à partir d'une plante contenant du safranal » ou « extrait végétal obtenu à partir d'une matière première végétale contenant du safranal », on entend au moins une molécule ou un ensemble de plusieurs molécules issue(s) de tout ou partie d'une plante contenant du safranal. Il peut s'agir d'une sélection spécifique de molécules natives présentes dans la plante ou de molécules obtenues par tout type de transformation desdites molécules natives. La matière première utilisée pour obtenir l'extrait peut être constituée par tout ou partie d'une plante contenant du safranal. Si la plante contenant le safranal est le safran, l'extrait végétal selon l'invention peut être obtenu en particulier à partir de stigmates et/ou de pétales et/ou de bulbes de safran.

L'extrait selon l'invention n'est pas la matière première en tant que telle. Il ne s'agit pas de plante, de partie de plante, de plante séchée, de partie de plante séchée ou d'échantillon de plante ou d'échantillon de plante séchée. Il ne s'agit pas non plus d'une huile essentielle. Par « plante contenant du safranal » ou « matière première contenant du safranal » (les termes plante et matière première végétale pouvant être utilisés indifféremment au sens de l'invention) on entend toute plante contenant du safranal en particulier *Crocus sativus, Centaurea sibthorpii, Centaurea consanguinea, Centaurea amanicola, Erodium cicutarium, Chinese green tea, Calycopteris floribunda, Crocus heuffelianus, Sambucus nigra, Gardenia jasminoides, Citrus limon, Cuminum cyminum L., Achillea distans.* Préférentiellement la plante contenant du safranal à partir de laquelle est obtenu l'extrait selon l'invention est *Crocus sativus* (safran).

L'extrait selon l'invention comprend au moins du safranal, et le safranal est présent à une concentration d'au moins 0,2% en poids de matière sèche, mesurée par méthode HPLC (Chromatographie Liquide Haute Performance). La méthode de mesure a toute son importance étant entendu qu'avec une autre méthode de mesure, notamment par spectrométrie UV (norme ISO 3632-2), le résultat obtenu ne correspond pas à la concentration réelle en safranal du fait de la non spécificité de cette méthode.

La méthode d'analyse par HPLC de molécules est une méthode connue de l'homme de l'art. Elle permet d'identifier et quantifier des molécules unitaires de façon précise. Préférentiellement, la méthode d'analyse utilisée pour doser les molécules contenues dans l'extrait selon l'invention, en particulier le safranal, est une méthode UHPLC (Chromatographie Liquide Ultra Haute Performance). Cette méthode permet d'augmenter encore la résolution et la séparation des composés, et de détecter plusieurs composés sur le même chromatogramme à partir d'un seul échantillon.

Selon un mode de réalisation particulièrement adapté, la méthode d'analyse HPLC ou UHPLC comprend une étape préalable de préparation de l'échantillon comprenant les étapes suivantes :
- Introduction de l'extrait de safran à doser dans une solution hydroalcoolique,
- Agitation magnétique pendant au moins 1 heure,
- Bain à ultrasons pendant au moins 5 minutes,
- Filtration sur membrane puis injection.

La méthode d'analyse, après préparation de l'échantillon comprend classiquement ensuite une étape d'élution puis de détection.

L'élution est préférentiellement réalisée à l'aide d'un gradient binaire. Le premier solvant peut être par exemple un solvant organique et un acide. Préférentiellement il s'agit de l'acide formique dans de l'acétonitrile. Le second solvant peut être par exemple un solvant aqueux acidifié. Préférentiellement il s'agit de l'acide formique dans l'eau.

En plus du safranal, l'extrait selon l'invention peut comprendre d'autres molécules, en particulier des crocines et/ou des flavonoïdes dérivés du kaempferol et/ou des dérivés de la picrocrocine, en particulier dans le cas où il s'agit d'un extrait de safran.

Dans le cas où elles sont présentes, les crocines représentent préférentiellement au moins 1% en poids de matière sèche de l'extrait, mesuré par méthode HPLC.

Dans le cas où ils sont présents, les flavonoïdes dérivés du kaempferol représentent préférentiellement au moins 500 ppm en poids de matière sèche de l'extrait, mesuré par méthode HPLC.

Dans le cas où elles sont présentes, les picrocrocines représentent préférentiellement au moins 0.5% en poids de matière sèche de l'extrait, mesurée par méthode HPLC.

L'extrait selon l'invention est préférentiellement imprégné sur un support. Par « support » ou « agent de charge » au sens de l'invention on entend toute substance alimentaire d'origine végétale, minérale ou chimique utilisée comme ingrédient ou additif alimentaire permettant l'imprégnation et par la même la dilution de l'extrait de l'invention.

Le support peut être notamment choisi parmi les constituants suivants : maltodextrine, sucre, silice, gomme arabique, préférentiellement la maltodextrine.

Lorsque l'extrait est imprégné sur un support, les pourcentages de molécules présentes dans l'extrait sont donnés en poids de matière sèche de l'extrait incluant le support.

L'extrait selon l'invention peut être obtenu par tout moyen permettant d'obtenir au moins 0,2% de safranal en poids de matière sèche de l'extrait. Préférentiellement l'extrait selon l'invention est obtenu par un procédé comprenant une étape de traitement thermique. Cette étape peut être mise en œuvre sur la matière première en début, en cours ou en fin de procédé d'obtention de l'extrait.

Par traitement thermique au sens de l'invention on entend chauffer à une température supérieure à la température ambiante. De façon préférée, l'étape de traitement thermique est un traitement thermique réalisé pendant au moins 2 heures, préférentiellement au moins 24 heures, à une température comprise entre 30°C et 95°C, préférentiellement entre 30°C et 60°C.

Le traitement thermique peut être réalisé par tout moyen connu, notamment en étuve, en four, par cuisson, pasteurisation ou débactérisation. Préférentiellement, le traitement thermique est réalisé en étuve.

Selon un mode de réalisation particulièrement adapté, l'extrait végétal selon l'invention est obtenu par un procédé comprenant la mise en œuvre des étapes suivantes réalisée à partir de matière première de safran :
- Eventuellement séchage,
- Broyage, préférentiellement entre 50 et 500 µm,
- Extraction aqueuse ou hydroalcoolique ou avec un solvant organique,
- Imprégnation sur un support de l'extrait obtenu,
- Traitement thermique.

Les étapes d'extraction aqueuse ou hydroalcoolique ou avec un solvant organique et d'imprégnation sur un support de l'extrait obtenu constitue le procédé d'extraction connu nommé Tech'Care Exctraction^{®}.

L'étape de traitement thermique peut être réalisé à tout moment de ce procédé, préférentiellement en fin de procédé sur la matière première de safran et complète le procédé Tech'Care Extraction^{®}.

Selon un mode de réalisation particulièrement adapté, l'étape de traitement thermique dans la mise en œuvre de ce procédé est une étape de traitement thermique dans une étuve pendant au moins 2 heures, encore plus préférentiellement pendant au moins 24 heures à une température comprise entre 30°C et 95°C, encore plus préférentiellement à une température comprise entre 30°C et 60°C.

Le broyage peut être réalisé par tout moyen adapté connu, notamment par un broyeur à couteau, à broches ou à marteau, préférentiellement un broyeur à broches.

L'étape d'extraction peut être réalisée par tout moyen adapté connu.

Dans le cas d'une extraction aqueuse, la matière broyée est introduite dans l'eau à raison de 50g/L.

Dans le cas d'une extraction hydroalcoolique, le solvant peut être notamment de l'éthanol, préférentiellement de l'éthanol à 60% v/v. La matière broyée est introduite dans la solution hydroalcoolique à raison de 50g/L.

Dans le cas d'une extraction avec un solvant organique, le solvant peut être notamment du méthanol ou de l'acétate d'éthyle, préférentiellement du méthanol à 30% v/v. La matière broyée est introduite dans le solvant organique à raison de 100g/L.

Après extraction, le procédé peut également comprendre une étape d'acidification. Cette étape consiste en l'ajout d'acide dans le solvant aqueux ou hydroalcoolique. Elle permet de diminuer le pH de la solution d'extraction entre 3 et 5. Elle peut être notamment réalisée dans les conditions suivantes : ajout d'acide citrique ou acide chlorhydrique dans le solvant hydroalcoolique pour ajuster le pH à 4.

L'étape d'imprégnation sur un support consiste en l'ajout d'un agent de charge dans la solution d'extraction. Le support ou agent de charge peut être notamment choisi parmi les constituants suivants : maltodextrine, sucre, silice, gomme arabique, préférentiellement la maltodextrine.

Après cette étape d'imprégnation, le procédé peut comprendre également une étape d'émulsion et/ou d'encapsulation de l'extrait obtenu. Cette étape consiste en l'agitation à haute vitesse de la solution d'extraction contenant l'agent de charge et éventuellement l'auxiliaire. Elle peut être notamment réalisée à l'aide d'auxiliaires tels que la gomme arabique, des cyclodextrines ou des matières grasses.

L'extrait selon l'invention peut être utilisé seul ou intégré dans une composition cosmétique, alimentaire, nutritionnelle ou médicamenteuse, à raison de 0,1 à 100% en poids de matière sèche de la composition. Préférentiellement, l'extrait selon l'invention est présent dans la composition en une quantité permettant l'administration chez l'homme ou l'animal d'au moins 0.07 mg d'extrait selon l'invention par kg de poids corporel par jour, encore plus préférentiellement entre 1.4 à 4.2 mg d'extrait selon l'invention par kg de poids corporel par jour.

L'invention a donc pour objet une telle composition, préférentiellement une composition se présentant sous forme de gélule, comprimé, capsule molle, stick, sachet, plat préparé, huile, lotion, crème, émulsion.

Les compositions comprenant un extrait selon l'invention peuvent contenir d'autres composants adaptés connus, tels que des excipients, sélectionnés en fonction de la forme et de l'utilisation envisagée de la composition ou d'autres principes actifs ou molécules actives. L'extrait selon l'invention et les compositions l'incluant peuvent être utilisés dans de nombreuses applications, en particulier pour prévenir ou traiter la dépression et l'anxiété ou encore pour prévenir ou traiter les troubles de l'humeur (troubles pathologiques de l'humeur), les troubles de l'érection, les troubles prémenstruels.

Aussi l'invention a également pour objet l'extrait pour son utilisation :
- dans la prévention ou le traitement de la dépression, chez l'être humain ou l'animal,
- dans la prévention ou le traitement de l'anxiété chez l'être humain ou l'animal,
- dans la prévention ou le traitement des troubles de l'humeur, chez l'être humain,
- dans la prévention ou le traitement des troubles de l'érection, chez l'être humain (chez l'homme),
- dans la prévention ou le traitement des troubles prémenstruels chez l'être humain (chez la femme).

Avantageusement, du fait de sa quantité importante en safranal, supérieure à celle de tous les extraits existants, l'extrait selon l'invention présente une grande efficacité pour ces applications. De plus, il est important d'utiliser un extrait plutôt que la matière première en tant que telle (plante en tout ou partie séchée ou non) car les extraits selon l'invention permettent d'augmenter la biodisponibilité des molécules actives car ces dernières ne sont plus imbriquées dans la matrice végétale de la fleur et sont plus accessibles à l'organisme. La présence du support dans l'extrait permet également d'obtenir une meilleure homogénéité des molécules d'intérêt dans le produit fini.

L'invention est à présent illustrée par des exemples d'extraits et procédés selon l'invention (exemples 1 et 2), d'exemples comparatifs d'extraits de l'art antérieur (exemples 3A et 3B) et de compositions.

Pour l'ensemble des exemples, la méthode de dosage des molécules dans l'extrait, et en particulier du safranal est une méthode UHPLC avec les caractéristiques suivantes :
1. Préparation de l'échantillon
Extraction de l'échantillon par une solution hydroalcoolique. Agitation magnétique d'au moins 1 heure puis bain à ultrasons pendant au moins 5 minutes. Filtration sur membrane puis injection.
2. Elution HPLC
Gradient binaire :
Solvant A (acide formique dans MeCN)
Solvant B (acide formique dans l'eau)
3. Détection

| | |
|---|---|
| Crocines | 440 nm |
| Dérivés de la picrocrocine | 250 nm |
| Flavonoïds | 350 nm |
| Safranal | 310 nm |

4. Standards
Trans-crocin-4-gentiobiose-gentobiose
Trans-crocin 3-gentiobiose-glucose
beta-cyclocitral
Kaempferol glucoside
Safranal

### Exemple 1 : Extrait selon l'invention

Un premier exemple d'extrait est un extrait obtenu par la mise en œuvre du procédé consistant en la mise en œuvre des étapes suivantes :
- utilisation de stigmates de Crocus sativus,
- broyage à l'aide d'un broyeur à broches, à une taille de 250µm,
- extraction hydroalcoolique à l'éthanol 60% v/v, à raison de 50g de safran par litre de solution hydroalcoolique,
- imprégnation sur maltodextrine, introduite dans la solution hydroalcoolique,
- traitement thermique à l'étuve pendant 48 heures à 40°C.

L'extrait obtenu est dosé en plusieurs molécules avec la méthode UHPLC décrite en préambule de la partie relative aux exemples.

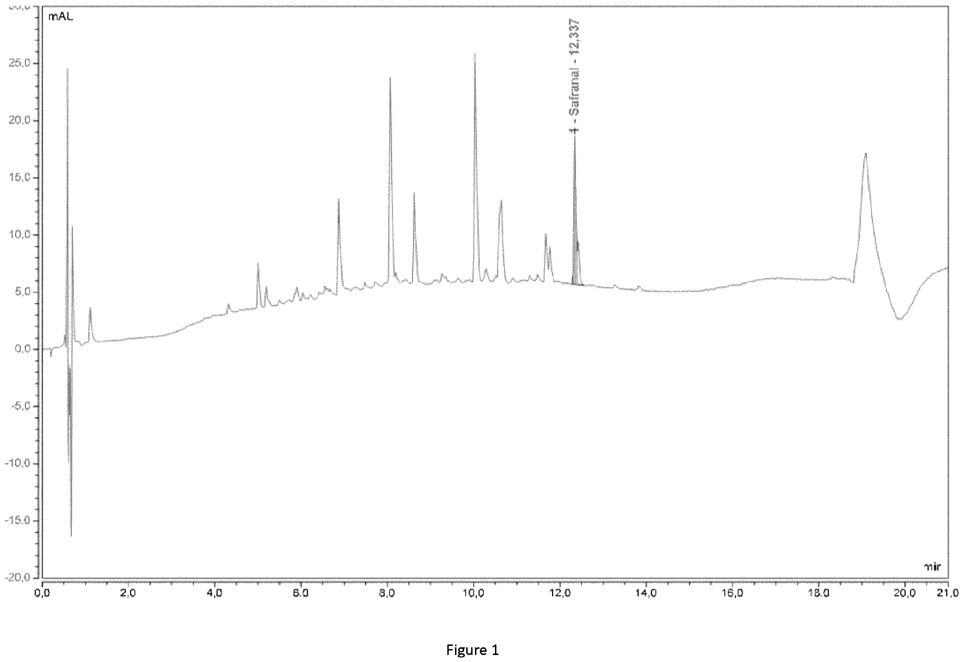

Le chromatogramme obtenu est présenté sur la Figure 1.

L'extrait est caractérisé par :
- une concentration en safranal de 0,238%,
- une concentration en Crocines de 3.96%,
- une concentration en dérivés de la picrocrocine de 1.08%, et
- une concentration en Flavonoïdes de 0.25%.

### Exemple 2 : Extrait selon l'invention

Un deuxième exemple d'extrait est un extrait obtenu par la mise en œuvre du procédé consistant en la mise en œuvre des étapes suivantes :
- utilisation de stigmates de Crocus Sativus,
- broyage à l'aide d'un broyeur à broches à une taille de 250 µm,
- extraction aqueuse acidifiée avec de l'acide chlorhydrique à pH 4,
- imprégnation sur gomme arabique introduite dans la solution aqueuse,
- traitement thermique à l'étuve pendant 72 heures à 40°C.

L'extrait obtenu est dosé en plusieurs molécules avec la méthode UHPLC décrite en préambule de la partie relative aux exemples.

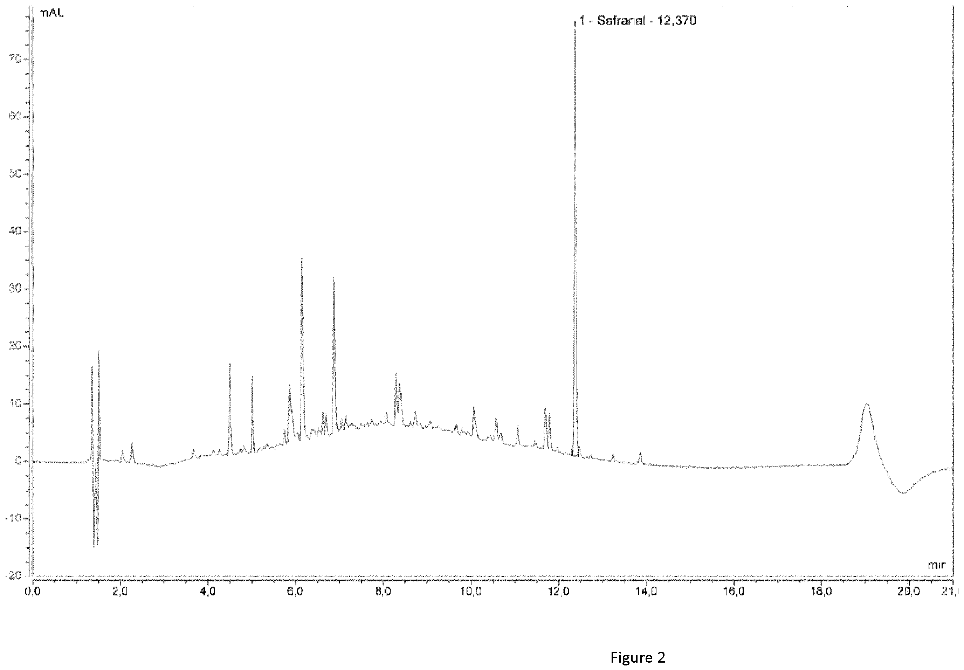

Le chromatogramme obtenu est présenté sur la Figure 2.

L'extrait est caractérisé par :
- une concentration en safranal de 0.73%,
- une concentration en Crocines de 1.0%,
- une concentration en dérivés de la picrocrocine de 2.93%, et
- une concentration en Flavonoïdes de 0.57%.

### Exemple 3 : Extrait selon l'invention

Un troisième exemple d'extrait est un extrait obtenu par la mise en œuvre du procédé consistant en la mise en œuvre des étapes suivantes :
- utilisation de stigmates de Crocus Sativus,
- broyage à l'aide d'un broyeur à broches à une taille de 250 µm,
- Premier traitement thermique à l'étuve de 2h à 6h à 105°C
- Deuxième traitement thermique à l'étuve de 2h à 6h à 140°C
- extraction hydroalcoolique à l'éthanol 60% v/v, à raison de 50g de safran par litre de solution hydroalcoolique,
- imprégnation sur maltodextrine, introduite dans la solution hydroalcoolique,
- séchage par lyophilisation de l'extrait liquide

L'extrait est caractérisé par :
- une concentration en safranal de 0.39%,
- une concentration en Crocines de 2.31%,
- une concentration en dérivés de la picrocrocine de 1.37%, et
- une concentration en Flavonoïdes de 0.24%.

### Exemple 3A : Extrait obtenu par atomisation (hors invention)

Un premier contre-exemple d'extrait est un extrait obtenu par la mise en œuvre du procédé consistant en la mise en œuvre des étapes suivantes :
- utilisation de stigmates de Crocus sativus,
- broyage à l'aide d'un broyeur à broches, à une taille de 250µm,
- extraction hydroalcoolique à l'éthanol 60% v/v, à raison de 50g de safran par litre de solution hydroalcoolique,
- imprégnation sur maltodextrine, introduite dans la solution hydroalcoolique,
- séchage par atomisation de l'extrait liquide.

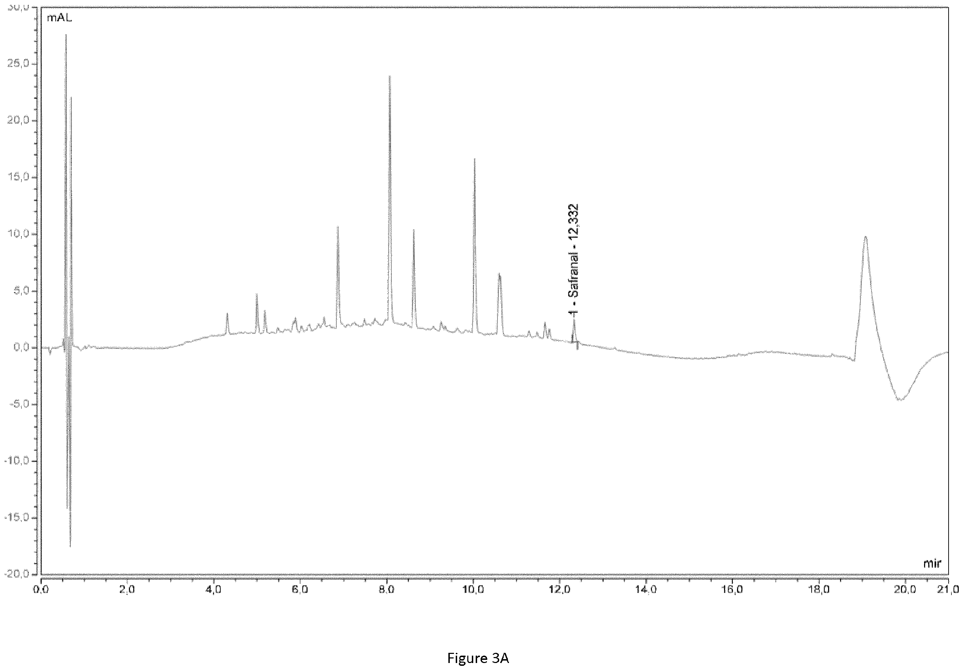

Le chromatogramme obtenu est présenté sur la Figure 3A.

L'extrait obtenu est caractérisé par :
- une concentration en safranal de 0,028%,
- une concentration en Crocines de 4,98%,
- une concentration en dérivés de la picrocrocine de 1.26%, et
- une concentration en Flavonoïdes de 0.24%

### Exemple 3B : Extrait obtenu par lyophilisation (hors invention)

Un deuxième contre-exemple d'extrait est un extrait obtenu par la mise en œuvre du procédé consistant en la mise en œuvre des étapes suivantes :
- utilisation de stigmates de Crocus sativus,
- broyage à l'aide d'un broyeur à broches, à une taille de 250u.m,
- extraction hydroalcoolique à l'éthanol 60% v/v, à raison de 50g de safran par litre de solution hydroalcoolique,
- imprégnation sur maltodextrine, introduite dans la solution hydroalcoolique,
- séchage par lyophilisation de l'extrait liquide.

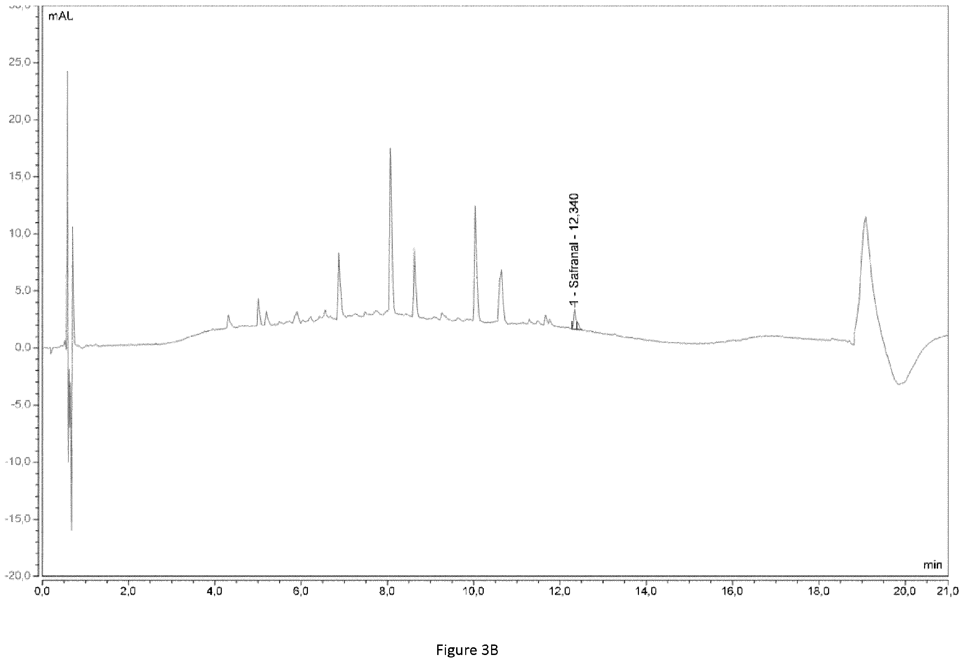

Le chromatogramme obtenu est présenté sur la Figure 3B.

L'extrait est caractérisé par :
- une concentration en safranal de 0,038%,
- une concentration en Crocines de 4.40%,
- une concentration en dérivés de la picrocrocine de 1.15%, et
- une concentration en Flavonoïdes de 0.22%.

### Exemple 4 : Exemple de composition nutritionnelle destinée à l'homme

L'exemple 4 est une gélule de 150 mg, constituée par :
- L'extrait selon l'invention de l'exemple 1 : 15mg
- Maltodextrine : 135mg

La composition est obtenue par mélange des constituants dans les conditions classiques connues de l'homme du métier, et mis dans une gélule selon les conditions classiques également.

La posologie préconisée est de 2 gélules par jour.

### Exemple 5 : Exemple de médicament destinée à l'homme

L'exemple 5 est un comprimé de 1500mg, constitué par :
- L'extrait selon l'invention de l'exemple 2 : 20mg,
- Sorbitol : 1430mg,
- Stéarate de magnesium : 27mg,
- Colorant bleu brillant FCF laque E133 : 20mg,
- Acesulfame K (E950) : 1,5mg,
- Saccharinate de sodium (E954) : 1,5mg.

La composition est obtenue par mélange des constituants dans les conditions classiques connues de l'homme du métier, et comprimée selon les conditions classiques également.

La posologie préconisée est de 1 comprimé par jour.

### Exemple 6 : Exemple de composition nutritionnelle destinée à l'animal

L'exemple 6 est un comprimé de 300 mg, constitué par :
- L'extrait de safran de l'exemple 1 : 6mg,
- Cellulose microcristalline : 135mg,
- Stéarate de magnésium : 10mg.

La composition est obtenue par mélange des constituants dans les conditions classiques connues de l'homme du métier, et comprimée selon les conditions classiques également.

La posologie préconisée est de 1 comprimé par jour.

### Exemple 7 : Composition cosmétique par voie topique sous forme de crème de jour

Il s'agit d'une composition se présentant sous forme de crème destinée à être appliquée sur la peau.

Elle est constituée par :
- L'extrait selon l'invention de l'exemple 1 : 10 mg,
- Conservateur : 0,5%,
- Parfums : 0,6%,
- Phase grasse + phase aqueuse : 97,4%.

La phase grasse est constituée d'un émulsionnant et de triglycérides. La phase aqueuse est constituée d'eau associée à un acide pyrrolidone carboxylique.

La composition est obtenue par ajout de l'extrait de safran de l'exemple 1, sous agitation en même temps que les conservateurs et les parfums à la phase aqueuse pendant 10minutes. La phase aqueuse est alors elle-même ajoutée sous agitation à la phase grasse pendant un mélange durant 30 minutes.

## Revendications

1. Extrait végétal obtenu à partir d'une matière première végétale contenant du safranal choisie parmi *Crocus sativus, Centaurea sibthorpii, Centaurea consanguinea, Centaurea amanicola, Erodium cicutarium, Chinese green tea, Calycopteris floribunda, Crocus heuffelianus, Sambucus nigra, Gardenia jasminoides, Citrus limon, Cuminum cyminum L., Achillea distans.,* **caractérisé en ce qu'**il comprend une concentration mesurée par méthode HPLC d'au moins 0,2% de safranal en poids par rapport au poids total de la matière sèche.

2. Extrait végétal selon la précédente revendication, **caractérisé en ce qu'**il comprend également des crocines et/ou des flavonoïdes dérivés du kaempferol et/ou des picrocrocines.

3. Extrait végétal selon l'une des précédentes revendications, **caractérisé en ce qu'**il est imprégné sur un support.

4. Extrait végétal selon la précédente revendications, **caractérisé en ce qu'**il est imprégné sur un support choisi parmi la maltodextrine, les sucres, la silice et la gomme arabique.

5. Extrait végétal selon l'une des précédentes revendications, **caractérisé en ce qu'**il est obtenu à partir de stigmates et/ou de pétales et/ou de bulbes de *Crocus sativus.*

6. Extrait végétal selon l'une des précédentes revendications, **caractérisé en ce qu'**il est susceptible d'être obtenu par un procédé comprenant une étape de traitement thermique d'au moins 2 heures à une température comprise entre 30°C et 95°C.

7. Extrait végétal selon la revendication 6, **caractérisé en ce que** la température est comprise entre 30°C et 60°C.

8. Extrait végétal selon l'une des revendications 6 ou 7, **caractérisé en ce que** le traitement thermique est réalisé au moins 24 heures.

9. Extrait végétal selon l'une des revendications 6 à 8, **caractérisé en ce que** le traitement thermique est réalisé en étuve, en four, par cuisson, pasteurisation ou débactérisation.

10. Extrait végétal selon l'une des revendications 6 à 9, **caractérisé en ce que** l'étape de traitement thermique peut être mise en œuvre sur la matière première en début, en cours ou en fin de procédé.

11. Procédé d'obtention d'un extrait végétal selon l'une des précédentes revendications, **caractérisé en ce qu'**il comprend la mise en œuvre des étapes suivantes :
- Broyage d'une matière première végétale contenant du safranal préférentiellement préalablement séchée,
- Extraction aqueuse ou hydroalcoolique ou avec un solvant organique,
- Imprégnation sur un support de l'extrait obtenu,
- Traitement thermique dans une étuve pendant au moins 2 heures à une température comprise entre 30°C et 95°C.

12. Procédé selon la revendications 11, **caractérisé en ce qu'**il comprend également une étape d'acidification après l'étape d'extraction.

13. Procédé selon la revendication 11 ou 12, **caractérisé en ce que** le séchage est réalisé pendant au moins 24 heures à une température comprise entre 30°C et 60°C.

14. Procédé selon l'une des revendications 11 à 13, **caractérisé en ce qu'**il comprend également une étape d'émulsion et/ou d'encapsulation de l'extrait obtenu.

15. Composition cosmétique, alimentaire, nutritionnelle ou médicamenteuse comprenant entre 0,1 et 100% en poids de matière sèche d'un extrait végétal selon l'une des revendications 1 à 9.

16. Composition selon la revendication 15, **caractérisé en ce qu'**elle se présente sous forme de gélules, comprimés, capsules molles, sticks, sachets, plats préparés, huile, lotion, crème, émulsion.

17. Extrait végétal selon l'une des revendications 1 à 10, pour son utilisation dans la prévention ou le traitement de la dépression, de l'anxiété chez l'être humain ou l'animal.

18. Extrait végétal selon l'une des revendications 1 à 10, pour son utilisation dans la prévention ou le traitement des troubles de l'humeur, des troubles de l'érection, des troubles prémenstruels chez l'être humain.
